Europäisches Patentamt

European Patent Office

Office européen des brevets

(11) Veröffentlichungsnummer : **0 341 466 B1**

(12) **EUROPÄISCHE PATENTSCHRIFT**

(45) Veröffentlichungstag der Patentschrift :
09.09.92 Patentblatt 92/37

(51) Int. Cl.⁵ : **C07C 205/26**

(21) Anmeldenummer : **89107202.7**

(22) Anmeldetag : **21.04.89**

(54) **Verfahren zur Bromierung von Nitroalkoholen.**

(30) Priorität : **30.04.88 DE 3814773**

(43) Veröffentlichungstag der Anmeldung :
**15.11.89 Patentblatt 89/46**

(45) Bekanntmachung des Hinweises auf die
Patenterteilung :
**09.09.92 Patentblatt 92/37**

(84) Benannte Vertragsstaaten :
**AT BE CH DE ES FR GB IT LI NL SE**

(56) Entgegenhaltungen :
DE-B- 1 768 976
CHEMICAL ABSTRACTS, Band 81, Nr. 19, 11.
November 1974, Seite 475, Spalte 2, Zusammenfassung Nr. 119937y, Columbus, Ohio,
USA& JP-A-74 70911
CHEMICAL ABSTRACTS, Band 89, Nr. 7, 14.
August 1978, Seite 553, Spalte 2, Zusammenfassung Nr. 59652e, Columbus, Ohio, USA

(56) Entgegenhaltungen :
CHEMICAL ABSTRACTS, Band 80, Nr. 11, 18.
März 1974, Seite 319, Spalte 1, Zusammenfassung Nr. 59447f, Columbus, Ohio, USA& JP-
A-73 072 108
PATENT ABSTRACTS OF JAPAN, Band 5, Nr.
194 (C-82)(866), 10. Dezember 1981 & JP-A-56
113 745

(73) Patentinhaber : Henkel
Kommanditgesellschaft auf Aktien
Postfach 1100 Henkelstrasse 67
W-4000 Düsseldorf 1 (DE)

(72) Erfinder : Wüst, Willi, Dr.
Fasanenring 32
W-4030 Ratingen (DE)
Erfinder : Eskuchen, Rainer, Dr.
Benrather Schlossallee 36
W-4000 Düsseldorf (DE)
Erfinder : Esser, Herbert
Siglarer Strasse 65
W-5210 Troisdorf (DE)

## Beschreibung

Die Erfindung betrifft ein Verfahren zur Bromierung von Nitroalkoholen an dem die Nitrogruppe tragenden Kohlenstoffatom. Nitroalkohole und Bromnitroalkohole sind an sich bekannte Substanzen. Ein wichtiger Vertreter ist beispielsweise 2-Brom-2-nitro-1,3-propandiol. Diese Verbindung hat als Konservierungsmittel und antimikrobieller Wirkstoff Bedeutung erlangt.

Zur Herstellung von Bromnitroalkoholen, bei denen Brom und Nitrogruppe am selben Kohlenstoff liegen, geht man üblicherweise von den Nitroalkoholen aus und setzt diese mit elementarem Brom um.

So wird in der japanischen Offenlegungsschrift 74/70911, zitiert bei Chem. Abstracts, Vol. 81, 119937y (1974), vorgeschlagen, durch Umsetzung von Nitromethan und Formaldehyd in Gegenwart von wäßriger Natronlauge 2-Nitro-1,3-propandiol herzustellen und dieses mit Brom in Dichlorethan zur entsprechenden Bromverbindung umzusetzen.

In der auf die Ammelderein zurückgehenden deutschen Auslegeschrift DE 17 68 976 wird bereits ein Verfahren zur Heerstellung von Brom-Nitroalkoholen beschrieben. Danach läßt man ein Nitroalkan mit einem Aldehyd in Gegenwart eines anorganischen Magnesium- oder Ezrdalkalisalzes und Wasser reagieren und bromiert die Erdalkalisalze der entsprechenden Nitroalkohole, ohne sie zu isolieren, in wäßriger Suspension bei einer Temperatur unterhalb von 25 °C. Nachteilig an diesem Verfahren ist die zusätliche Mitverwendung von erdalkalisalzen, die hinterher entsorgt werden müssen.

Das spanische Patent ES 453 224 schlägt die Herstellung von Brom-Nitroalkoholen durch Bromierung von Nitroalkoholen in einem Zweiphasen-System Wasser/Methylenchlorid in Gegenwart von Phasentransfer-Katalysatoren vor. Für einen technishen Prozeß ist bei dieser Vorgehensweise jedoch ein Nachteil in der Mitverwendung eines organischen Lösungsmittels, das entsorgt oder wieder eingesetzt werden muß, zu sehen.

Die japanischen Patentanmeldung JP-A-73/72108 (C.A. 80:59447f (1974)) schlägt in ähnlicher Weise vor, die durch Kondensation von Nitro-Alkanen mit Aldehyden entstandenen Nitro-Alkohole als Erdalkalisalze direkt zu bromieren. Es wird jedoch Brom zu der Salzlösung hinzugetropft. Nachteilig an dieser Vorgehensweise ist jedoch, daß im Vergleich zu der weiter unten zu diskutierenden Erfindung mehr Nebenprodukte entstehen.

Auch die JP-A-56/113745 (Patent Abstracts of Japan, Band 5, Nr. 194 (c-82 (866)) betrifft ein Verfahren zu Herstellung Brom-Nitroalkoholen. Nach dieser Patentanmeldung wird in Gegenwart eines Alkanpolyols, wie beispielsweise Diethylenglykol gearbeitet. Das Verfahren ist daher praktisch nur für solche Verwendungen der Brom-Nitroalkohole geeignet, bei denen diese gelöst in einem Alkanpolyol eingesetzt werden sollen, da die Aufarbeitung ansonsten schwierig ist.

Vor dem Hintergrund dieses Standes der Technik ist es Aufgabe der Erfindung, zur Durchführung derartiger Bromierungsreaktionen ein Verfahren vorzuschlagen, das ohne die Verwendung organischer Lösungsmittel auskommt, und die gewünschten Bromnitroalkohole in hoher Raumzeitausbeute und hoher theoretischer Ausbeute herzustellen. Dabei hat sich gezeigt, daß es von Vorteil ist, den Nitroalkohol in wäßriger Lösung und in Salzform einem Brom/Bromwasserstoffgemisch zuzudosieren.

Es wurde erstaunlicherweise festgestellt, daß sich die Bromierungsreaktion in technischem Maßstab bei hohen Konzentrationen und damit hohen Raumzeitausbeuten durchführen läßt, indem man die Salzlösung in die Brom/Bromwasserstoff-Lösung-Mischung einleitet. Bei umgekehrter Zugabereihenfolge entstehen im wesentlichen Umfang Nebenprodukte.

Gegenstand der Erfindung ist somit ein Verfahren zur Herstellung von geminalen Brom-nitro-alkoholen durch Bromierung von Alkalioder Erdalkalimetallsalzen von Nitro-alkoholen, wobei man eine Mischung von Brom und wäßriger Bromwasserstofflösung über einen Wärmetauscher im Kreislauf pumpt und vor dem Wärmetauscher die wäßrige Lösung des Alkali- oder Erdalkalimetallsalzes eines Nitro-Alkohols in einer solchen Geschwindigkeit unter Kühlung zudosiert, daß die maximale Reaktionstemperatur 30°C nicht übersteigt.

Als Mischung von Brom und Bromwasserstofflösung werden erfindungsgemäß Mischungen von Brom mit einer Bromwasserstofflösung von 50 bis 62 Gew.-% HBr, insbesondere azeotrop siedende wäßrige HBr-Lösung eingesetzt. Das Mischungsverhältnis (molar) beträgt dabei 1 : 3 bis 1 : 10, vorzugsweise 1 : 5 bis 1 : 5,5 und insbesondere um 1 : 5,3.

In diese Mischung werden erfindungsgemäß die Alkalimetallsalze oder auch, soweit, wasserlöslich, die Erdalkalimetallsalze der Nitroalkohole eingeführt. Umgesetzt werden können Alkali- oder Erdalkalimetallsalze zahlreicher bekannter Nitroalkohole. Bevorzugt sind die Alkalimetallsalze von Nitroalkoholen, die durch Umsetzung von Nitroparaffinen mit 1 - 8 C-Atomen, aber insbesondere von Nitromethan mit kurzkettigen Aldehyden, wie Formaldehyd, Acetaldehyd, Propionaldehyd oder auch Chloral erhalten werden können, nach der Maßgabe, daß sie ein acides Wasserstoffatom an dem die Nitrogruppe tragenden C-Atom aufweisen.

Nach einer bevorzugten Ausführungsform des Verfahrens werden Alkali- oder Erdalkalimetallsalze des 2-Nitro-1,3-propandiols eingesetzt. Unter diesen Salzen ist das Kaliumsalz bevorzugt. Wäßrige Lösungen dieses Kaliumsalzes können beispielsweise nach der deutschen Patentanmeldung DE 38 14 772(internes Zeichen des Anmelders D 8191) eingesetzt werden.

Um das erfindungsgemäße Verfahren mit hohen Raumzeitausbeuten durchführen zu können, is es bevorzugt, die Erdalkalimetallsalze in 40 bis 60 Gew.-%iger Wäßriger Lösung oder Suspension einzusetzen.

Erfindungsgemäß wird die beim Eintropfen des Salzes in die Mischung aus Brom und Bromwasserstofflösung entstehende Reaktionswärme über Wärmetauscher abgeführt. Hierbei eignen sich Plattenwärmetauscher oder vorzugsweise Rohrbündelwärmetauscher. Auch anders konstruierte Wärmetauscher sind einsetzbar, wobei generell gilt, daß mit fallender Austauschfläche auch die Eintropfgeschwindigkeit gesenkt werden muß, da bei der Reaktion eine Temperatur von 30°C vorteilhafterweise nicht überschritten werden sollte.

Nach einer bevorzugten Ausführungsform des Verfahrens wird die Mischung aus Brom und Bromwasserstofflösung in einem Rührkessel aus korrosionsfestem Werkstoff, beispielsweise Tantal, hergestellt und über eine korrosionsfesten Rohrreaktor mit Hilfe einer Kreiselpumpe in den Rohrbündelwärmetauscher gepumpt und von dort in den Kessel zurückgeleitet. Die Reaktionsmischung wird so lange umgepumpt, bis die gesamte Lösung des Salzes des Nitroalkohols eingeleitet worden ist.

Nach einer bevorzugten Ausführungsform der Erfindung wird, falls am Reaktionsende noch Brom vorhanden ist, was sich an einer Färbung der Lösung zeigt, dieses durch Eingeben eines Reduktionsmittels, beispielsweise von Hydroxylaminlösung, reduziert. Dazu wird die Hydroxylaminlösung nach und nach zugetropft, bis sich der Reaktionsansatz zumindest weitgehend entfärbt hat.

Die nach dem erfindungsgemäßen Verfahren primär erhaltene Mischung von Bromiden und 2-Brom-2-nitro-alkoholen kann entweder in üblicher Weise aufgearbeitet werden, wozu der 2-Brom-2-nitro-alkohol mit geeigneten Lösungmitteln extrahiert werden kann, oder aber sie wird als solche in einem nachfolgenden Reaktionsschritt weiter verarbeitet.

Beispielsweise kann 2-Brom-2-nitro-1,3-propandiol ohne Zwischenisolierung direkt zu 5-Brom-5-nitro-1,3-dioxan umgesetzt werden.

2-Brom-2-nitro-alkohole sind wichtige Konservierungsmittel bzw. Mikrobizide. Darüber hinaus ist das 2-Brom-2-nitro-1,3-propandiol als Vorstufe für das vorgenannte 5-Brom-5-nitro-1,3-dioxan, welches ebenfalls als mikrobizider Wirkstoff eingesetzt wird, von Bedeutung.

Beispiele

Beispiel 1

Herstellung von 2-Nitro-propandiol-1,3-kaliumsalz

Gearbeitet wurde in einer Technikumsanlage, bestehend aus einem 0,8 m³ Rührwerksbehälter, einer Kreiselpumpe mit einem Durchsatz von 25 m³·h⁻¹; H = 40 m und einem 12 m² Plattenwärmetauscher.

In dem Rührwerksbehälter werden 229,2 kg 37 Gew.-%ige wässrige Formaldehydlösung vorgelegt und durch Umpumpen über den Wärmetauscher zurück in den Rührwerksbehälter auf eine Temperatur von 0 bis 10 °C gekühlt. Innerhalb von 30 min wurden dann 201,4 kg einer 45 Gew.-%igen wässrigen Kalilauge zudosiert, wobei die Vermischung im Schergefälle der Kreiselpumpe eintrat. Die Temperatur der Mischung wurde im Bereich von 0 bis 10 °C gehalten.

Zu dieser Mischung wurden innerhalb von 2 Stunden 82,1 kg Nitromethan zudosiert. Dabei wurde die Dosiergeschwindigkeit so eingestellt, daß die Temperatur der Reaktionsmischung an keiner Stelle 15 °C überstieg. Die Temperatur war über den Dosierstrom des Nitromethans regelbar. Die Nachreaktionszeit betrug 15 min, während denen das Reaktionsgemisch im Kreis gepumpt wurde. Am Ende der Reaktionszeit war der Restgehalt an Nitromethan auf unter 1 Gew.-% gefallen. Es konnte gezeigt werden, daß das gebildete Kaliumsalz des 2-Nitro-1,3-propandiols bei Temperaturen bis hinab zu -15 °C in Lösung blieb. Durch Neutralisation der Salzlösung ließ sich 2-Nitro-1,3-propandiol herstellen.

Beispiel 2

Bromierung des 2-Nitro-1,3-propandiol-Kaliumsalzes

Die Bromierung wurde in einem emaillierten Rührwerksbehälter mit einem Volumen von 1,3 m³ durchgeführt. Dieser war über eine Rohrleitung mit einer Kreiselpumpe mit einem Durchsatz von 10 m³/h, H = 12 m, mit einem 6 qm Rohrbündelwärmetauscher verbunden. Der Auslauf des Wärmetauschers wurde wiederum mit dem Rührwerksbehälter verbunden. Aus einem Brom-Lagerbehälter wurden 228,3 kg Brom und aus einem weiteren Lagerbehälter 35,1 kg 62 %ige HBr in den Rührwerksbehälter gegeben und durch den Produkt-Kühlkreislauf gepumpt. Innerhalb von 1,5 h wurden dann 512,7 kg der Reaktionsmischung aus Beispiel 1 (Kaliumsalz des 2-Nitro-1,3-propandiols) zudosiert. Die Reaktionstemperatur wurde wärend der gesamten Reaktionszeit unter 30°C gehalten; bei Erreichen der 30°C wurde der Dosierstrom der Kaliumsalzlösung gedrosselt. Nach beendeter Zugabe und nach einer Nachreaktionszeit von 0,5 h wurde das überschüssige Brom mit 14,8 kg einer 40 Gew.-%igen Hydroxylammoniumchlorid-Lösung innerhalb von 0,25 h reduziert.

## Patentansprüche

1. Verfahren zur Herstellung von geminalen Brom-nitro-alkoholen durch Bromierung von Alkali- oder Erdalkalimetallsalzen von Nitro-alkoholen, dadurch gekennzeichnet, daß man eine Mischung von Brom und wäßriger Bromwasserstofflösung über einen Wärmetauscher im Kreislauf pumpt und vor dem Wärmetauscher die wäßrige Lösung des Alkali- oder Erdalkalimetallsalzes eines Nitro-Alkohols in einer solchen Geschwindigkeit unter Kühlung zudosiert, daß die maximale Reaktionstemperatur 30°C nicht übersteigt.

2. Verfahren nach Anspruch 1, dadurch gekennzeichnet, daß als Mischung von Brom und Bromwasserstoff-Lösung eine Mischung von Brom mit wäßriger Bromwasserstoff-Lösung eines Bromwasserstoffgehalts zwischen 50 und 62 Gew.-% einsetzt, wobei das molare Mischungsverhältnis $Br_2$ : HBr = 1 : 3 bis 1 : 10, vorzugsweise 1 : 5 bis 1 : 5,5, beträgt.

3. Verfahren nach einem der Ansprüche 1 oder 2, dadurch gekennzeichnet, daß man als Alkalimetallsalze Kaliumsalze einsetzt.

4. Verfahren nach einem der Ansprüche 1 bis 3, dadurch gekennzeichnet, daß man als Alkalimetallsalz des Nitroalkohols 2-Nitro-1,3-propandiol-Kaliumsalz einsetzt.

5. Verfahren nach den Ansprüchen 1 bis 4, dadurch gekennzeichnet, daß man das Alkalimetallsalz des Nitroalkohols in Form einer 40 bis 60 Gew.-%igen wäßrigen Lösung einsetzt.

6. Verfahren nach einem der Ansprüche 1 bis 5, dadurch gekennzeichnet, daß man als Wärmetauscher eine Plattenwärmetauscher oder einen Rohrbündelwärmetauscher einsetzt.

7. Verfahren nach einem der Ansprüche 1 bis 6, dadurch gekennzeichnet, daß man am Reaktionsende einen etwaigen Überschuß an Brom durch Zugabe von Hydroxylaminlösung bis zur zumindest weitgehenden Entfärbung des Reaktionsansatzes reduziert.

## Claims

1. A process for the production of geminal bromonitro alcohols by bromination of alkali metal or alkaline earth metal salts of nitro alcohols, characterized in that a mixture of bromine and aqueous hydrogen bromide solution is pump-circulated through a heat exchanger and, before the heat exchanger, an aqueous solution of the alkali metal or alkaline earth metal salt of a nitro alcohol is added with cooling at such a rate that the maximum reaction temperature does not exceed 30°C.

2. A process as claimed in claim 1, characterized in that a mixture of bromine with aqueous hydrogen bromide solution having a hydrogen bromide content of 50 to 62% by weight is used as the mixture of bromine and hydrogen bromide solution, the molar ratio of $Br_2$ to HBr being 1:3 to 1:10 and preferably 1:5 to 1:5.5.

3. A process as claimed in claim 1 or 2, characterized in that potassium salts are used as the alkali metal salts.

4. A process as claimed in any of claims 1 to 3, characterized in that 2-nitropropane-1,3-diol potassium salt is used as the alkali metal salt of a nitro alcohol.

5. A process as claimed in claims 1 to 4, characterized in that the alkali metal salt of a nitro alcohol is used in the form of a 40 to 60% by weight aqueous solution.

6. A process as claimed in any of claims 1 to 5, characterized in that a plate-type heat exchanger or a tube bundle heat exchanger is used as the heat exchanger.

7. A process as claimed in any of claims 1 to 6, characterized in that, at the end of the reaction, any excess bromine is reduced by addition of hydroxylamine solution until the reaction mixture is at least substantially colorless.


**Revendications**

1°) Procédé de production de nitroalcools bromés géminés par bromation de sels de métaux alcalins ou alcalino-terreux de nitroalcools, caractérisé en ce qu'on envoie en circuit par pompage sur un échangeur thermique un mélange de brome et d'une solution aqueuse de bromure d'hydrogène et en ce qu'en amont de l'échangeur thermique on ajoute par dosage en refroidissant la solution aqueuse du sel métallique alcalin ou alcalino-terreux d'un nitro-alcool à une vitesse telle, que la température maxima de la réaction ne dépasse pas 38°C.

2°) Procédé selon la revendication 1, caractérisé en ce qu'on utilise comme mélange de brome et de solution de bromure d'hydrogène un mélange de brome avec une solution aqueuse de bromure d'hydrogène contenant entre 50 et 62% en poids de bromure d'hydrogène, le rapport molaire du mélange $Br_2 : HBr$ se situant de 1 : 3 jusqu'à 1 : 10, de préférence de 1 : 5 à 1 : 5,5.

3°) Procédé selon une des revendications 1 ou 2, caractérisé en ce qu'on utilise des sels de potassium comme sels de métaux alcalins.

4°) Procédé selon une des revendications à 3, caractérisé en ce qu'on utilise comme sel de métal alcalin du nitro-alcool, du sel de potassium du 2-nitro-1,3-propanediol.

5°) Procédé selon les revendications 1 à 4, caractérisé en ce qu'on utilise le sel de métal alcalin du nitro-alcool sous la forme d'une solution aqueuse à 40 - 60% en poids.

6°) Procédé selon une des revendications 1 à 5, caractérisé en ce qu'on utilise comme échangeur thermique un échangeur à plaques ou un échangeur à faisceau de tubes.

7°) Procédé selon une des revendications 1 à 6, caractérisé en ce qu'à la fin de la réaction on réduit un éventuel excédent de brome par addition de solution d'hydroxylamine au moins jusqu'à décoloration importante du produit de réaction.